# EUROPÄISCHE PATENTSCHRIFT

(11) **EP 2 379 211 B1**
(45) Veröffentlichungstag und Bekanntmachung des Hinweises auf die Patenterteilung: **13.02.2013**
(21) Anmeldenummer: 09760699.0
(22) Anmeldetag: 02.12.2009
(51) Int. Cl.: B01F 11/00, B01F 13/00, B01F 13/06, B01F 15/00, B01F 15/02, A61F 2/30, A61F 2/46

(54) **MISCHVORRICHTUNG MIT ANZEIGE FÜR DRUCKÄNDERUNG**
MIXING APPARATUS HAVING DISPLAY FOR PRESSURE CHANGE
DISPOSITIF MÉLANGEUR AVEC INDICATION DE CHANGEMENT DE PRESSION

(30) Priorität: 21.01.2009 CH 89092009
(43) Veröffentlichungstag der Anmeldung: 26.10.2011
(73) Patentinhaber: Medmix Systems AG, 6343 Rotkreuz (CH)
(72) Erfinder: GRETER, Andy, CH-6340 Baar (CH); VEID, Martin, CH-6353 Weggis (CH); STÖCKLI, Rochus, CH-6374 Buochs (CH); KELLER, Wilhelm, A., CH-6402 Merlischachen (CH)
(74) Vertreter: Detken, Andreas
(86) Internationale Anmeldenummer: PCT/CH2009/000384
(87) Internationale Veröffentlichungsnummer: WO 2010/083616

(56) Entgegenhaltungen:
- EP-A- 1 525 864
- EP-B- 1 076 776
- WO-A-97/07748
- WO-A-99/06140
- WO-A-2005/018831
- WO-A-2008/009143
- DE-U1- 20 005 333
- US-A1- 2003 155 381

## Beschreibung

### TECHNISCHES GEBIET

Die vorliegende Erfindung betrifft eine Mischvorrichtung zum Mischen wenigstens zweier Komponenten, insbesondere eine Mischvorrichtung zum Mischen von Produkten für medizinische Anwendungen, wie etwa Knochenzement.

### STAND DER TECHNIK

In verschiedenen technischen Gebieten ist es erforderlich, ein Produkt, das zur Weiterverarbeitung vorgesehen ist, erst unmittelbar vor seiner Anwendung aus verschiedenen Komponenten durch Mischen zu erzeugen. Dies kann erforderlich sein, da das Produkt sofort nach der Mischung seinen Zustand ändert, z. B. aushärtet oder in verschiedene Bestandteile zerfällt. In der Medizinaltechnik sind derartige Produkte z. B. bei Pharmazeutika bekannt, die in ihrem Anwendungszustand nicht gelagert werden können, oder sind z. B. aus der Herstellung von medizinischen Klebstoffen oder Knochenzement bekannt. Das Produkt wird in der Regel aus wenigstens zwei Komponenten zusammengesetzt, wobei meist eine Komponente als Feststoff und eine weitere Komponente als Flüssigkeit vorliegt. Kurz vor der Anwendung wird die Flüssigkeit mit dem Feststoff vermischt und das so entstandene Verarbeitungsprodukt wird unmittelbar an seinem Bestimmungsort verarbeitet.

Aus der WO 97/07748A1 ist beispielsweise ein derartiges Mischsystem bekannt. Eine feste Komponente ist in einem ersten Behälter untergebracht, an den ein zweiter Behälter angeschlossen werden kann und dabei eine Flüssigkeitsverbindung herstellt. In den zweiten Behälter kann beispielsweise eine Ampulle mit einer flüssigen Komponente eingesetzt werden. Der zweite Behälter kann relativ zum ersten Behälter verdreht werden, wodurch ein Öffnungsmechanismus betätigt wird, welcher die Ampulle in dem zweiten Behälter öffnet, so dass die flüssige Komponente aus der Ampulle über die Flüssigkeitsverbindung in den ersten Behälter geleitet werden kann. Zur Unterstützung der Umleitung der Flüssigkeitskomponente kann an das Behältersystem ein Vakuum angelegt werden, das über einen Anschlussstutzen in dem ersten Behälter erzeugt werden kann. Durch den Druckunterschied zwischen dem Innenraum der Ampulle und dem Vakuum im Behältersystem wird sichergestellt, dass die Flüssigkeit vollständig aus der Ampulle entleert wird.

Bei der Mischvorrichtung nach dem Stand der Technik kann das Anliegen des Vakuums an dem Behältersystem kontrolliert werden, indem das Ein- oder Ausschalten der Einrichtung zur Vakuumerzeugung überwacht wird. Ist die Vakuumeinrichtung eingeschaltet, sollte davon ausgegangen werden können, dass zumindest in dem ersten Behälter ein Vakuum anliegt. Es kann jedoch nicht überprüft werden, ob auch in dem zweiten Behälter oder gar der Ampulle ein Vakuum erzeugt wurde. Es könnte sein, dass die Flüssigkeitsverbindung blockiert ist und somit die Vakuumerzeugung im zweiten Behälter nicht stattfinden kann. Ferner kann nicht überprüft werden, ob die Ampulle tatsächlich geöffnet wurde und somit die flüssige Komponente durch das Vakuum in den ersten Behälter gelangen konnte.

In US 2003/0155381A1 ist in den Figuren 13-16 ebenfalls eine Mischvorrichtung offenbart, bei der eine Monomerkomponente unter Vakuumanwendung aus einem Komponentenbehälter in eine Mischvorrichtung transportiert wird. Die Monomerkomponente ist in einer Glasampulle aufgenommen. Die Glasampulle befindet sich in einem Ampullenhalter und ist darin in der Längsrichtung verschiebbar. Am unteren Ende des Ampullenhalters ist ein Keil vorhanden, auf den die Ampullenspitze aufschiebbar ist, um diese abzubrechen. Oberhalb der Ampulle ist ein Kolben angeordnet, der dichtend im Ampullenhalter geführt ist. Um die Monomerkomponente in den Mischbehälter zu transferieren, wird im Mischbehälter und dadurch auch im Ampullenhalter ein Vakuum erzeugt. Durch den Luftdruck wird der Kolben des Ampullenhalters vertikal nach unten gezogen und presst die Ampulle auf den Keil, wodurch die Ampullenspitze abbricht. Anschliessend wird die Monomerkomponente durch das Vakuum aus der Ampulle herausgesaugt. Indem die Position des Kolbens beobachtet wird, kann festgestellt werden, ob tatsächlich ein Vakuum anliegt.

Während diese Vorrichtung an sich erlaubt, das Vorliegen eines Vakuums im Ampullenhalter festzustellen, ist es nicht ganz einfach, diese Feststellung zweifelsfrei zu treffen, da der Kolben durch das Vakuum in den Ampullenhalter hinein bewegt wird und dadurch bei korrekter Funktion nicht mehr sichtbar ist. Der Benutzer ist also gezwungen, indirekt auf die korrekte Funktion zu schliessen, indem er feststellt, dass der Kolben nicht mehr sichtbar ist. Dies ist nachteilig, da der Kolben auch aus anderen Gründen nicht sichtbar sein könnte, z.B. weil vergessen wurde, den Kolben einzusetzen. Dadurch ist die Funktionssicherheit der Vorrichtung beeinträchtigt.

Die WO 99/06140 A1 offenbart eine Mischvorrichtung gemäss dem Oberbegriff des Anspruchs 1, wobei die Anzeigeeinrichtung als flexible Indikatorscheibe ausgebildet ist. Bei Anwendung eines Vakuums wird die Indikatorscheibe durch den Luftdruck nach innen verformt.

Es ist eine Aufgabe der vorliegenden Erfindung, eine Mischvorrichtung zum Mischen wenigstens zweier Komponenten vorzusehen, bei der das Vorliegen einer Druckänderung im Mischsystem oder/und das Entleeren einer Komponentenkammer in eine Mischkammer zuverlässig überprüft werden kann, die einfach in der Handhabung ist und eine einfache Bauweise aufweist.

### DARSTELLUNG DER ERFINDUNG

Diese Aufgabe wird von der Erfindung durch eine Mischvorrichtung nach Anspruch 1 gelöst. Vorteilhaft Ausgestaltungen und weitere Ausführungsformen gehen aus den abhängigen Ansprüchen hervor.

Eine Mischvorrichtung zum Mischen wenigstens zweier Komponenten nach der vorliegenden Erfindung weist eine Mischkammer mit einer ersten Komponente und wenigstens eine Komponentenkammer mit einer weiteren, von der ersten verschiedenen Komponente auf, die mit der Mischkammer über eine Zuleitung verbunden ist oder verbunden werden kann. Die Mischkammer ist vorzugsweise zylindrisch ausgebildet und ist mit der ersten Komponente z. B. bis zur Hälfte des Volumens gefüllt. Ist als Mischprodukt beispielsweise Knochenzement vorgesehen, kann in der Mischkammer die pulverförmige Komponente und in der wenigstens einen Komponentenkammer kann die Mischflüssigkeit vorgesehen sein. Die Mischkammer mit der Pulverkomponente und die Komponentenkammer können getrennt von einander dauerhaft gelagert werden. Vorzugsweise ist in der Mischkammer ein relativ zur Mischkammer beweglicher Mischer vorgesehen. Der Mischer kann beispielsweise als Rührelement ausgebildet sein, das an einer Kolbenstange befestigt ist, die im Inneren der Mischkammer in Längsrichtung der Kammer beweglich gelagert ist. Die Mischkammer ist dann vorzugsweise an einem Ende durch einen Stopfen abgeschlossen, in dem die Kolbenstange des Mischers gleitend geführt sein kann. Am gegenüberliegenden Ende der Mischkammer ist dann eine Öffnung vorgesehen, an welche sich die Zuleitung für die Komponentenkammer anschliesst. In gelagertem Zustand kann die Mischkammer verschlossen sein und erst vor der Herstellung der Mischung geöffnet und an die die Zuleitung angeschlossen werden. An der Öffnung der Mischkammer kann ein Filter oder Sieb vorgesehen werden.

Im Fall der Herstellung eines herkömmlichen Knochenzements ist es ausreichend, neben der Mischkammer eine einzige Komponentenkammer vorzusehen. Grundsätzlich ist es jedoch auch möglich, mehr als eine Komponentenkammer mit jeweils einer weiteren oder auch der gleichen Komponente wie in der ersten Komponentenkammer über eine Zuleitung an die Mischkammer anzuschliessen. Die Komponentenkammer ist vorzugsweise ebenfalls zylindrisch ausgebildet. Die weitere Komponente kann dabei entweder unmittelbar in der Komponentenkammer untergebracht sein oder sie kann in einem Komponentenbehälter vorgesehen sein, der in die Komponentenkammer eingesetzt werden kann.

Ferner ist in der Mischvorrichtung eine Unterdruckeinrichtung, insbesondere eine Vakuumeinrichtung, vorgesehen, die in der Mischkammer einen Unterdruck erzeugen kann. Wenn die Unterdruckeinrichtung aktiviert ist, liegt somit in der Mischkammer ein geringerer Druck an, als in der Komponentenkammer. Vorzugsweise wird in der Mischkammer annähernd ein Vakuum erzeugt. Die Unterdruckeinrichtung ist hierfür über einen Anschluss mit der Mischkammer verbunden, der vorzugsweise in einem Bereich der Kammer liegt, in dem sich keine Komponentenpartikel befinden. Bei einer aufgestellten zylindrischen Mischkammer befindet sich beispielsweise die erste Komponente in einem unteren Teil der Kammer und der Anschluss für die Unterdruckeinrichtung in einem oberen Teil der Kammer. Beim Aktivieren der Unterdruckeinrichtung breitet sich der Unterdruck von der Mischkammer durch die Zuleitung bis in die Komponentekammer, bzw. die Komponentenkammern, aus.

Durch die Aktivierung der Unterdruckeinrichtung wird die weitere Komponente aus der wenigstens einen Komponentenkammer u. a. aufgrund des Druckausgleichs zwischen der Komponentenkammer und der Mischkammer in die Mischkammer transportiert, bzw. gesaugt. Eine flüssige Komponente kann auch zumindest teilweise durch den Ausgleich eines Höhenunterschieds der Flüssigkeitssäulen in der Mischkammer und der Komponentenkammer von der Komponentenkammer in die Mischkammer transferiert werden.

Es ist möglich, aber nicht notwendig, eine Verschlusseinrichtung vorzusehen, die in einer Ausgangsstellung der Mischvorrichtung gemäss der Erfindung die Zuleitung zwischen der Mischkammer und der wenigstens einen Komponentenkammer, bzw. die Zuleitung zu der Komponente, verschliesst. Die Verschlusseinrichtung kann z. B. durch ein Ventil in der Zuleitung gegeben sein, das z. B. durch manuelle Betätigung von einer geschlossenen in eine geöffnete Stellung gebracht werden kann. Die Verschlusseinrichtung kann aber auch mit einem in der Komponentenkammer untergebrachten Komponentenbehälter gekoppelt sein. Der Komponentenbehälter ist derart in der Komponentenkammer gelagert, dass er in seinem verschlossenen Zustand die Zuleitung verschliesst, bzw. blockiert, und in seinem geöffneten Zustand die Zuleitung frei gibt. Das Öffnen der Verschlusseinrichtung ist somit direkt an das Öffnen des Komponentenbehälters in der Komponentenkammer gekoppelt. Ist der Komponentenbehälter beispielsweise durch eine Ampulle gegeben, kann die Verschlusseinrichtung z. B. durch Abtrennen des Ampullenkopfes geöffnet werden. Ferner kann die Verschlusseinrichtung auch unmittelbar durch die Komponente in der Komponentenkammer gebildet werden. Durch die Komponente wird ein Teil des Volumens der Komponentenkammer abgetrennt, so dass der Teil der Kammer vor der Komponente zur Zuleitung offen und der Teil der Kammer hinter der Komponente von der Zuleitung getrennt ist.

Zum Öffnen des Komponentenbehälters z. B. in Form einer Ampulle kann z. B. an der Komponentenkammer eine Öffnungseinrichtung vorgesehen sein. Die Öffnungseinrichtung kann sich im Wesentlichen im Inneren der Komponentenkammer befinden, aber von einer Aussenseite der Komponentenkammer betätigt werden. Die Öffnungseinrichtung ist vorzugsweise gemäss WO 2010/012114 A1 ausgebildet. Es ist jedoch auch möglich, andere Einrichtungen zum Öffnen des Komponentenbehälters, wie sie beispielsweise auch aus dem Stand der Technik bekannt sind, zu verwenden.

Gemäss der vorliegenden Erfindung ist an der Mischvorrichtung an wenigstens einer der Komponentenkammern eine Anzeigevorrichtung vorgesehen, die eine Druckänderung in der Komponentenkammer anzeigt. Durch die erfindungsgemässe Anzeigevorrichtung kann das Anliegen eines von der Unterdruckeinrichtung erzeugten Unterdrucks in der Komponentenkammer überwacht werden. Gleichzeitig kann somit überprüft werden, dass die Zuleitung geöffnet ist. Vorzugsweise wird mit der Anzeigevorrichtung eine Druckminderung in der Komponentenkammer überprüft. Somit kann festgestellt werden, dass durch einen Druckausgleich zwischen dem niedrigeren Druck in der Mischkammer und dem höheren Druck in der Komponentenkammer die weitere Komponente aus der Komponentenkammer in die Mischkammer transportiert, bzw. gesaugt wurde. Die Anzeige dient somit ebenfalls als Überwachung der Entleerung der Komponentenkammer.

Die Anzeigevorrichtung wird durch einen elastischen Bereich der Komponentenkammer gebildet. So kann die Kammer z.B. durch einen Zylinder aus festem Material gebildet sein, der nur in einem vorbestimmten Bereich ein elastisches Material aufweist. Der elastische Bereich kann z.B. in der Umfangswand des Zylinders vorgesehen sein, ist vorzugsweise jedoch an einem Ende als Abschluss des Zylinders angeordnet. Vorzugsweise liegt dieses Ende dem anderen Zylinderende mit der Offnung zur Zuleitung zur Mischkammer gegenüber. Der elastische Bereich wird durch einen Balg gebildet, der an dem Zylinderende aufgesetzt werden kann. Der Balg kann als Faltenbalg oder auch als Ballonbalg ausgebildet sein. Bei einer Druckänderung in der Komponentenkammer zieht sich der Balg im Falle einer Druckminderung zusammen und im Falle einer Druckerhöhung wird der Balg aufgebläht.

In der Ausgangsstellung der Mischvorrichtung befindet sich die Anzeigevorrichtung in einer ersten Position. Durch Aktivieren der Unterdruckeinrichtung kann ein Druckunterschied zwischen der Mischkammer und der wenigstens einen Komponentenkammer erzeugt werden. Nachdem ein Druckausgleich, d. h. eine Druckminderung, in der Komponentenkammer erfolgt ist, befindet sich die Anzeigevorrichtung in einer zweiten Position, die angibt, dass in der Komponentenkammer eine Druckänderung stattgefunden hat. Die Anzeigevorrichtung wechselt somit durch die Druckänderung von einer Ausgangsstellung zu einer Anzeigestellung. Vorzugsweise umfasst die Anzeigevorrichtung eine Farbmarkierung zur farblichen Unterscheidung der Ausgangsstellung von der Anzeigestellung. Grundsätzlich ist es jedoch auch möglich, die Stellungen durch Buchstaben, Ziffern oder andere Zeichen zu markieren.

Zur Herstellung der gewünschten Mischung mit einer erfindungsgemässen Mischvorrichtung kann zunächst die Komponente aus der Komponentenkammer zumindest teilweise in die Zuleitung und auch in die Mischkammer eingeführt werden, beispielsweise durch Öffnen einer Ampulle in der Komponentenkammer oder durch Öffnen einer Verschlusseinrichtung zwischen Zuleitung und Komponentenkammer. Die Zuleitung ist somit durch die Komponente verschlossen. Anschliessend kann mittels der Unterdruckeinrichtung ein Unterdruck in der Mischkammer relativ zur Komponentenkammer erzeugt werden. Durch den Druckausgleich wird zunächst die in der Zuleitung und der Komponentenkammer verbliebene Komponente in die Mischkammer gesaugt und anschliessend erfolgt ein Druckausgleich in der Komponentenkammer, der die Anzeigevorrichtung aktiviert, d. h. von der Ausgangsstellung in die Anzeigestellung versetzt.

Alternativ kann bei der Mischvorrichtung nach der Erfindung auch zuerst in der Mischkammer ein Unterdruck erzeugt werden, sofern eine Verschlusseinrichtung vorgesehen ist, welche die Zuleitung zur Komponentenkammer, bzw. den Teil der Komponentenkammer mit der Anzeigeeinrichtung, blockiert. Durch Öffnen der Verschlusseinrichtung kann dann zum einen die Komponente aus der Komponentenkammer in die Mischkammer transferiert und zum anderen ein Druckausgleich zwischen Mischkammer und Komponentenkammer ermöglicht werden, wobei die Anzeigevorrichtung von der Ausgangsstellung in die Anzeigestellung versetzt wird.

Sobald die Anzeigevorrichtung von der Ausgangsstellung in die Anzeigestellung zur Anzeige der Druckänderung gewechselt ist, kann der Anwender sicher sein, dass die wenigstens eine Komponentenkammer geleert ist und die weitere Komponente durch die Zuleitung in die Mischkammer übergeführt wurde. Er kann nun mit dem Mischer der Mischkammer die Komponenten miteinander vermischen. Anschliessend kann auf die Mischkammer über der Öffnung eine Austragdüse aufgesetzt werden und die Mischung beispielsweise mittels Vorschub eines Stopfens in der Mischkammer durch die Austragdüse ausgetragen werden.

Die Anzeigevorrichtung ist vorzugsweise derart ausgebildet, dass eine Volumenänderung des Volumens der Komponentenkammer, an der die Anzeigevorrichtung vorgesehen ist, angezeigt werden kann. Im Falle einer Druckminderung in der Kammer durch den Druckausgleich zwischen der Mischkammer und der Komponentenkammer erfolgt bei dieser Ausführungsform gleichzeitig eine Volumenverkleinerung der Komponentenkammer.

In bevorzugten Ausführungsformen ist die Anzeigevorrichtung an einem Ende der Komponentenkammer angeordnet, das dem Ende, an das sich die Zuleitung anschliesst, gegenüber liegt. Die Komponentenkammer weist somit an einem Ende die Anzeigevorrichtung auf und an einem diesem Ende gegenüberliegenden Ende einen Auslass, der in die Zuleitung zur Mischkammer mündet. Zwischen der Anzeigevorrichtung und der Zuleitung ist vorzugsweise die Komponente in der Kammer vorgesehen. Durch diese Ausführungsform der Anzeigevorrichtung ist sichergestellt, dass eine Druckänderung über das gesamte Volumen der Komponentenkammer vorliegt. Sobald ein Druckausgleich zwischen der Mischkammer und der Komponentenkammer erfolgt, wird sich dieser über das gesamte Volumen der Komponentenkammer erstrecken und somit auch die Betätigung der Anzeigevorrichtung auslösen.

Bevorzugt ist die Anzeigevorrichtung entgegen einer Elastizitätskraft von der Ausgangsstellung in die hierzu unterschiedliche Anzeigestellung beweglich. Die Elastizitätskraft kann beispielsweise durch die Verformung eines Materials oder durch eine in der Anzeigevorrichtung vorgesehene Federeinrichtung erzeugt werden. Bei einer Druckminderung in der Komponentenkammer muss somit die Elastizitätskraft überwunden werden, um die Anzeigevorrichtung von der Ausgangsstellung in die Anzeigestellung bewegen zu können. Sobald nach einer Druckänderung und einer damit verbundenen Bewegung der Anzeigevorrichtung von der Ausgangstellung in die Anzeigestellung ein Druckausgleich mit dem Umgebungsdruck erfolgt, wird die Anzeigevorrichtung durch die Vorspannung der Elastizitätskraft von der Anzeigestellung wieder zurück in die Ausgangsstellung bewegt. Dies ist beispielsweise möglich, wenn die Unterdruckeinrichtung deaktiviert wird oder wenn die Komponentenkammer von dem Gesamtsystem aus Mischkammer, Zuleitung und Komponentenkammer getrennt wird, so dass durch die Kammeröffnung ein Druckausgleich mit der Umgebung erfolgen kann. Die Elastizitätskraft des Materials oder der Federeinrichtung bildet eine Gegenkraft entgegen der Kraft, die bei einer Druckänderung in der Komponentenkammer an der Anzeigevorrichtung angreift. Geringfügige Druckänderungen in der Komponentenkammer rufen daher noch keine Veränderung der Anzeigevorrichtung hervor, da dessen Kraft nicht ausreicht, um die Elastizitätskraft zu überwinden.

Vorzugsweise ist gemäss der vorliegenden Erfindung die Anzeigevorrichtung abnehmbar an der Komponentenkammer vorgesehen. Dadurch kann vorteilhafterweise ein Zugang zur Komponentenkammer geschaffen werden, um beispielsweise die weitere Komponente oder einen Komponentenbehälter mit der weiteren Komponente in der Kammer unterzubringen. Anschiessend kann die Anzeigevorrichtung wieder an der Komponentenkammer angebracht werden. Ist die Anzeigevorrichtung z. B. durch einen elastischen Balg gegeben, kann dieser beispielsweise mit seiner Öffnung aufgeweitet werden, um ihn über eine Öffnung an dem Komponentenzylinder zu stülpen. In aufgesetztem Zustand zieht sich die Öffnung des Balgs soweit zusammen, dass eine luftdichte Verbindung zwischen der Komponentenkammer und dem Balg entsteht. In entspanntem Zustand befindet sich der Balg dann in einer Ausgangsstellung und kann durch eine Druckänderung entgegen seiner Elastizitätskraft in eine Anzeigestellung bewegt werden. Bei einer Ausführungsform einer Mischvorrichtung nach der vorliegenden Erfindung ist eine Basiseinheit vorgesehen, in der zumindest ein Teil der Zuleitung vorgesehen ist und die Anschlüsse zum Anschliessen der Mischkammer und der wenigstens einen Komponentenkammer an die Zuleitung, bzw. den Teil der Zuleitung, aufweist. Die Mischkammer und die Komponentenkammer sind vorzugsweise lösbar an den Anschlüssen angeordnet. Die Mischkammer, die wenigstens eine Komponentenkammer und die Basiseinheit bilden somit einen Bausatz, der die Mischvorrichtung bildet. Ein Anwender kann somit zum Mischen von wenigstens zwei Komponenten die Mischkammer und die wenigstens eine Komponentenkammer über den Anschlüssen der Basiseinheit anbringen und die Mischkammer an die Unterdruckeinrichtung anschliessen. Durch Aktivieren der Unterdruckeinrichtung kann somit durch den Druckausgleich zwischen der Komponentenkammer und der Mischkammer die zweite Komponente in die Mischkammer geleitet werden. Die Komponenten können mittels des beweglichen Mischers gemischt, die Mischkammer kann von der Basiseinheit abgenommen werden und die Mischung ist bereit für eine Anwendung.

### BESCHREIBUNG DER ZEICHNUNGEN

Bevorzugte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen stellen dar:
- Fig. 1: einen Längsschnitt durch eine erste Ausführungsform einer Mischvorrichtung nach der vorliegenden Erfindung in einer Ausgangsstellung;
- Fig. 2: einen Längsschnitt durch die Ausführungsform nach Figur 1 mit geöffneter Verschlusseinrichtung;
- Fig. 3: einen Längsschnitt der Ausführungsform nach Figur 1 in einer Anzeigestellung; und
- Fig. 4: einen Längsschnitt durch eine zweite Ausführungsform einer Mischvorrichtung nach der vorliegenden Erfindung.

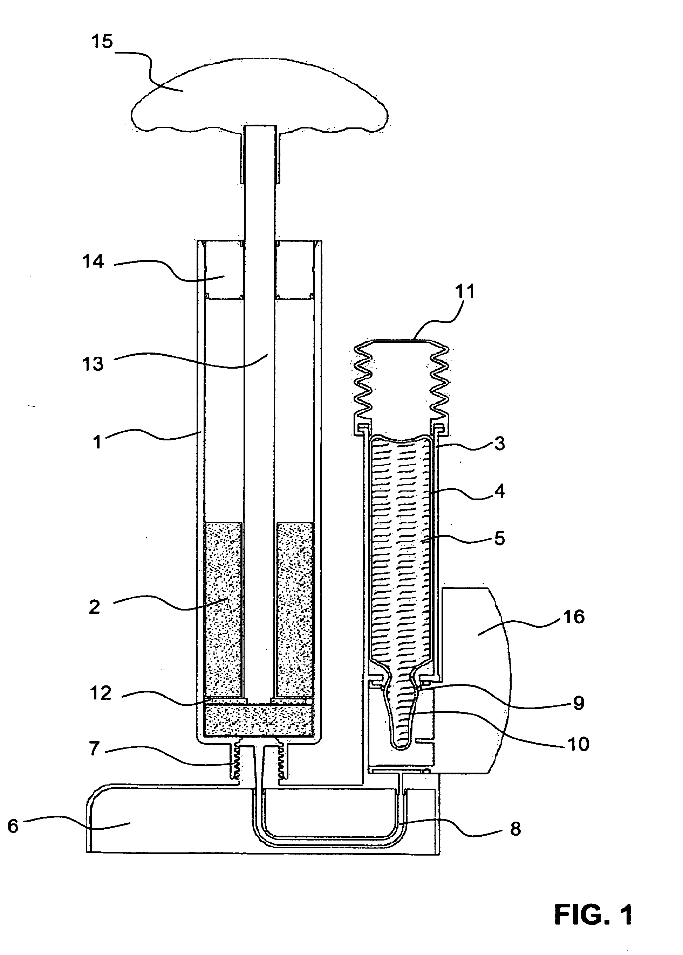

In Figur 1 ist eine erste Ausführungsform einer Mischvorrichtung zum Mischen wenigstens zweier Komponenten gemäss der vorliegenden Erfindung gezeigt. Die Mischvorrichtung weist eine Mischkammer 1 mit einer ersten Komponente 2 und eine Komponentenkammer 3 auf, in der ein Komponentenbehälter 4 mit einer zweiten Komponente 5 untergebracht ist. Die Mischkammer 1 und die Komponentenkammer 3 sind auf einer Basiseinheit 6 befestigt. Die Mischkammer 1 ist lösbar an der Basiseinheit 6 mittels einer Schraubverbindung 7, in diesem Fall einem Bajonettverschluss, angebracht.

Die Komponentenkammer 3 ist in dem gezeigten Ausführungsbeispiel fest mit der Basiseinheit verbunden und im vorliegenden Fall einstückig mit der Basiseinheit ausgebildet. Alternativ ist es jedoch auch möglich, auch die Komponentenkammer lösbar mit der Basiseinheit zu verbinden. Die Komponentenkammer 3 ist mit der Mischkammer 1 über eine Zuleitung 8 verbunden. Die Zuleitung 8 verläuft durch das Basisteil 6 und mündet an der Oberseite der Basiseinheit aus der Basiseinheit. Die Zuleitung erstreckt sich in die Schraubverbindung 7 und stellt mit der Mischkammer 1 in aufgesetztem Zustand der Mischkammer eine Fluidverbindung zum Inneren der Mischkammer her. Am anderen Ende mündet die Zuleitung 8 in das Innere der Komponentenkammer 3. Über der Öffnung der Mischkammer 1 zur Zuleitung 8 kann ein Filter vorgesehen sein, der die erste Komponente 2, etwa ein Pulver, in der Mischkammer zurückhalten kann. Über der Öffnung der Komponentenkammer 3 zur Zuleitung 8 kann ein weiterer Filter vorgesehen sein, der Verunreinigungen oder Splitter, die beispielsweise durch das Öffnen eines Komponentenbehälters in Form einer Ampulle entstehen, zurückzuhalten kann. Die Mischvorrichtung ist in einer Ausgangsstellung gezeigt, in der die Komponenten in ihren jeweiligen Kammern getrennt von einander gelagert sind.

An der Rückseite der Mischkammer gemäss Figur 1 weist die Mischkammer im oberen Teil einen Anschlussstutzen (nicht gezeigt) für eine Unterdruckeinrichtung auf, die in der Mischkammer relativ zur Komponentenkammer einen Unterdruck erzeugen kann.

Im Inneren der Komponentenkammer 3 wird der Komponentenbehälter 4 in Form einer Ampulle durch flexible Lippen 9 gehalten, die an einem nach Innen ragenden Flansch der Komponentenkammer vorgesehen sind. Die flexiblen Lippen 9 stehen in einem unteren Bereich nahe des Auslasses der Komponentenkammer 3 zur Zuleitung 8 in das Innere der Komponentenkammer 3 vor. Der Komponentenbehälter 4 weist an einem Ende einen Behälterkopf 10 auf, mit dem er durch die flexiblen Lippen 9 der Komponentenkammer hindurchragt und von diesem gehalten wird. Die flexiblen Lippen 9 können zum Beispiel aus elastischem Material, wie etwa Gummi, ausgebildet sein, das an dem Behälterkopf 10 um seinen gesamten Umfang anliegt und somit den Komponentenbehälter 4 in der Komponentenkammer 3 tragt. In der Ausgangsstellung gemäss Figur 1 befindet sich die Mischvorrichtung in einer Ruheposition, in der sie auch über einen längeren Zeitraum aufbewahrt werden kann.

Es ist denkbar, dass die flexiblen Lippen 9 als Dichtlippen wirken und eine Art Verschlusseinrichtung zwischen der Mischkammer und der Komponentenkammer bilden. Die flexiblen Lippen 9 unterteilen die Komponentenkammer in zwei Bereiche, einen Bereich vor der Verschlusseinrichtung und einen Bereich hinter der Verschlusseinrichtung in dem sich der Ampullenkörper befindet. In der Ausgangsstellung gemäss Figur 1 können die flexiblen Lippen 9 dicht an dem Behälterkopf 10 anliegen, so dass die Verschlusseinrichtung in einer verschlossenen Position ist.

An der Komponentenkammer 3 ist eine Anzeigevorrichtung in Form eines elastisch verformbaren Balgs 11 vorgesehen, der eine Druckänderung in der Komponentenkammer 3 anzeigen kann. Der Balg 11 befindet sich an einem offenen Ende des Zylinders der Komponentenkammer 3, das dem Ende, welches der Zuleitung 8 zugewandt ist, gegenüberliegt. Der Balg 11 ist mit einer Öffnung über die Öffnung der Komponentenkammer 3 gestülpt und schliesst die Öffnung der Komponentenkammer dicht ab. Der Balg 11 ist ziehharmonikaartig ausgebildet, d. h. er weist auf seiner Aussenumfangsfläche eine zickzack-artige, vorgegebene Faltung auf, entlang derer der Balg in Längsrichtung zusammengestaucht werden kann, wobei die Falten aufeinander zu liegen kommen. In der in Figur 1 gezeigten Stellung ist der Balg in einer entspannten Position, d. h. in der Ausgangsstellung.

In der Mischkammer 1 ist ein relativ zur Mischkammer beweglicher Mischer 12 vorgesehen. Der Mischer 12 ist an einer Kolbenstange 13 angebracht, die an dem Ende der Mischkammer 1, das dem Ende in Richtung der Zuleitung 8 gegenüberliegt, aus der Mischkammer 1 herausragt. Die Kolbenstange 13 ist beweglich in einem Stopfen 14 gelagert, der die Mischkammer an diesem Ende dicht abschliesst. Sobald die zweite Komponente 5 in die Mischkammer 1 umgeleitet wurde, kann die Kolbenstange 13 an einem Griff 15 ergriffen werden und der Mischer 12 durch Ein- und Ausschieben und Verdrehen der Kolbenstange 13 in und aus der Mischkammer 1 bewegt werden, um die Komponenten miteinander zu mischen. Das Mischen erfolgt vorzugsweise in aufgesetztem Zustand der Mischkammer 1 auf die Basiseinheit 6. Die Mischkammer 1 kann aber auch vor dem Mischen von der Basiseinheit 6 abgenommen werden, wobei zum Mischen die Öffnung 7 wieder verschlossen werden muss.

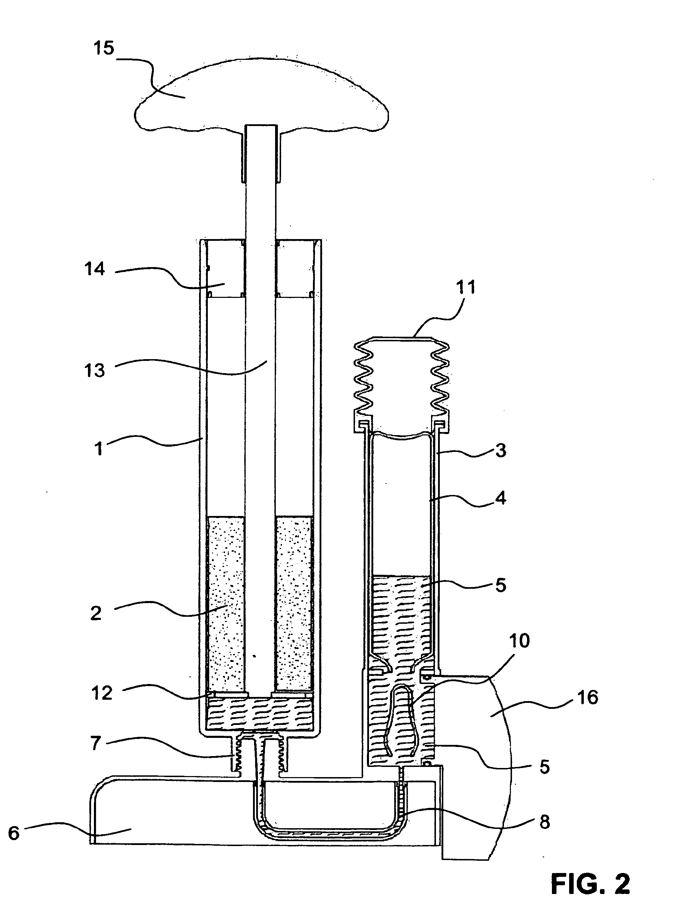

In Figur 2 ist die Mischvorrichtung nach Figur 1 in einem Zustand gezeigt, in dem der Komponentenbehälter 4 geöffnet wurde. Hierfur ist seitlich an der Komponentenkammer 3 ein Drehhebel 16 angeordnet, der mit einer Öffnungseinrichtung im Inneren der Komponentenkammer 3 derart verbunden ist, dass durch ein Drehen des Drehhebels 16 der Behälterkopf 10 vom Komponentenbehälter 4 abgetrennt wird. In Figur 2 ist der Drehhebel 16 in einer nach unten gedrehten Position gezeigt, in der der Behälterkopf 10 abgetrennt wurde, so dass die zweite Komponente 5 aus dem Komponentenbehälter 4 austritt Bezüglich der Ausgestaltung der Öffnungseinrichtung für den Komponentenbehälter 4 wird auf die Beschreibung der schon erwähnten WO 2010/012114 A1 verwiesen. Bei der Ausgestaltung des Drehhebels 16 ist darauf zu achten, dass der Übergang zur Komponentenkammer 3 abgedichtet ist.

Durch das Öffnen des Komponentenbehälters 4 fliesst ein Teil der Komponente 5 in die Zuleitung 8 und teilweise bereits in die Mischkammer 1. Der Transfer erfolgt u. a. durch die unterschiedliche Höhe der Flussigkeitssäulen der Komponente 5 in der Komponentenkammer 3 und der Mischkammer 1. In dem Kompönentenbehalter 4 ist über der Komponente 5 ein Restgasvolumen vorgesehen. Beim Öffnen des Komponentenbehälters 4, bzw. beim Abbrechen des Behälterkopfes 10, expandiert das Gas, so dass aufgrund der Expansion ein Druck auf die Komponente 5 ausgeübt wird und diese in die Zuleitung 8 gedrängt wird.

Nach dem Öffnen des Komponentenbehälters kann die Unterdruckeinrichtung aktiviert werden, wodurch in der Mischkammer 1 relativ zur Kompönentenkammer 3 ein Unterdruck erzeugt wird. Es erfolgt ein Druckausgleich zwischen der Kompönentenkammer 3 mit höherem Druck und der Mischkammer 1 mit niedrigerem Druck, so dass in der Komponentenkammer 3 eine Druckänderung, respektive eine Druckminderung, erfolgt. Mit dem Druckausgleich wird der Rest der zweiten Komponente 5 durch die Zuleitung 8 in die Mischkammer 1 befördert. Dabei strömt die zweite Komponente auch aus dem Inneren des Komponentenbehalters 4 und aus dem Behälterkopf 10, der mit der Öffnung nach unten von der Öffnungseinrichtung gehalten wird, durch die Zuleitung 8 in die Mischkammer 1.

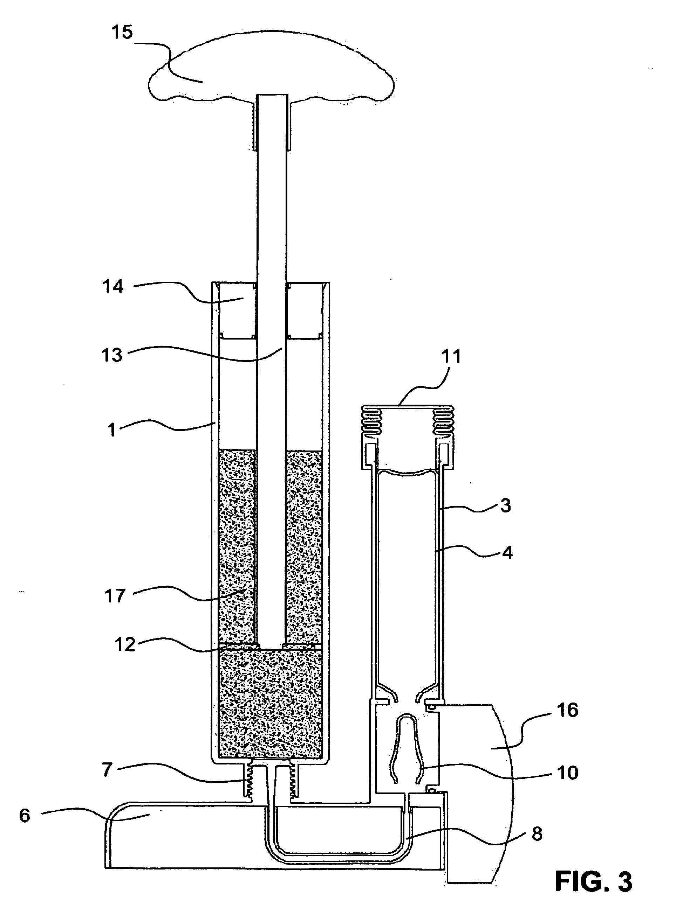

In Figur 3 ist die zweite Komponente 5 vollständig von der Komponentenkammer 3 in die Mischkammer transferiert worden. Der Unterdruck, der durch die Unterdruckeinrichtung an der Mischkammer 1 angelegt wird, erstreckt sich über die Zuleitung 8 bis in die Komponentenkammer 3 und den Balg 11. Aufgrund der elastischen Eigenschaften des Balgs 11 wird dieser durch das Anliegen des Unterdrucks in seinem Inneren, im Vergleich zum Umgebungsdruck, komprimiert. Die Kompression des Balgs dient als Anzeige für die Druckänderung in der Komponentenkammer 3. Durch die Kompression des Balgs 11 verringert sich das Gesamtvolumen der Komponentenkammer 3.

Der Balg kann beispielsweise auf der Aussenseite in den Flächen zwischen den Falten eine rötliche Färbung aufweisen und auf der Aussenseite an den Spitzen der Falten und der Oberseite des Balgs eine grünliche Färbung aufweisen. In expandiertem Zustand des Balgs gemäss Figur 1 sind die roten Flächen zwischen den Falten ersichtlich, so dass die Ausgangsstellung angezeigt wird. Im komprimierten Zustand des Balgs werden die roten Flächen zwischen den Falten durch das Aufeinanderlegen der Falten abgedeckt und lediglich die an den Spitzen der Falten vorgesehene grünliche Färbung ist ersichtlich, wodurch die Druckänderung angezeigt werden kann.

Die Mischung 17 aus den Komponenten 2 und 5 in der Mischkammer 1 kann durch Bewegung des Mischers 12 mit Hilfe des Griffs 15 hergestellt werden. Die Mischkammer 1 kann nun von der Basiseinheit 6 abgenommen werden. An dem Teil der Gewindeverbindung der Mischkammer 1 kann anschliessend eine Austragdüse angebracht werden und die Mischung 17 kann aus der Mischkammer 1 in bekannter Weise ausgetragen werden.

Bei einer altemaiven Vorgehensweise kann mittels der Unterdruckeinrichtung auch bereits vor dem Öffnen des Komponentenbehälters 4 ein Unterdruck in der Mischkammer 1 angelegt werden. In diesem Fall können z. B. die flexiblen Lippen 9 als Verschlusseinrichtung dienen. In der Ausgangsstellung gemäss Figur 1 erzeugt die Unterdruckeinrichtung im Inneren der Mischkammer 1, in der Zuleitung 8 und im Bereich der Komponentenkammer 3, der vor den flexiblen Lippen 9 liegt, bzw. der Zuleitung zugewandt ist, einen Unterdruck. Dabei herrscht in der Komponentenkammer 3 auf der gegenüberliegenden Seite der flexiblen Lippen 9, d. h. hinter den flexiblen Lippen, im Komponentenbehälter 4 und im Volumen des Balgs 11 ein höherer Druck (in der Regel der Umgebungsdruck). Es besteht somit vor und hinter der Verschlusseinrichtung in Form der an dem Behälterkopf anliegenden flexiblen Lippen ein unterschiedlicher Druck.

In Figur 2 befindet sich die Verschlusseinrichtung in einem geöffneten Zustand, da die Dichtung zwischen den flexiblen Lippen 9 und dem Behälterkopf 10 durch das Abtrennen des Behälterkopfs 10 von dem Komponentenbehälter 4 aufgelöst wurde. Es kann ein Druckausgleich im gesamten im Komponentenbehälter 4 und im Volumen des Balgs 11 erfolgen, wodurch die Anzeigevorrichtung von einer Ausgangsstellung in eine Anzeigestellung gebracht wird.

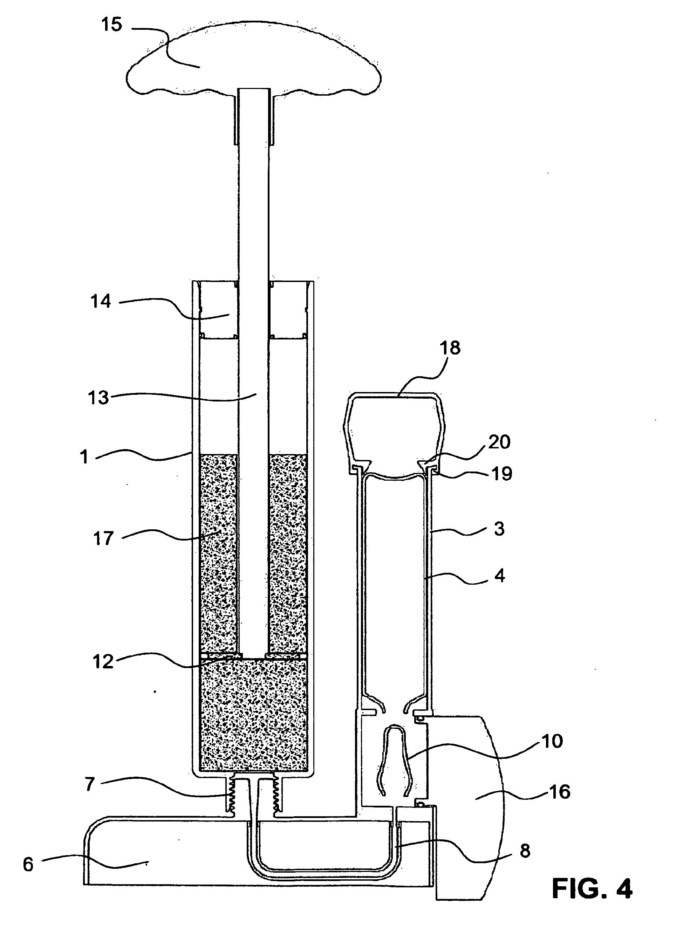

In Figur 4 ist eine zweite Ausführungsform einer Mischvorrichtung nach der vorliegenden Erfindung gezeigt. Gleiche Bauteile werden mit gleichen Bezugszeichen versehen. In dieser Ausführungsform ist auf der Öffnung der Komponentenkammer 3, welche der Öffnung für die Zuleitung 8 gegenüberliegt, ein Ballonbalg 18 als Anzeigevorrichtung aufgesetzt. Der Ballonbalg 18 weist eine Öffnung auf, an deren Umfang eine äussere Dichtlippe 19 und eine innere Dichtlippe 20 vorgesehen ist, zwischen welcher der Umfangsrand der Komponentenkammer 3 in montiertem Zustand zu liegen kommt. Dabei schliesst die äussere Dichtlippe 19 des Ballonbalgs 18 an der Aussenseite der Komponentenkammer 3 und die innere Dichtlippe 20 an der Innenseite der Komponentenkammer 3 an. Eine vergleichbare Befestigung kann auch für den Faltenbalg 11 vorgesehen werden. Der Ballonbalg 18 ist bauchförmig ausgebildet und besteht aus derart elastischem Material, dass sich seine Umfangswandung nach Innen in das Innere des Ballonsvolumens krümmen kann. In Figur 4 ist der Ballonbalg jedoch in expandiertem Zustand gezeigt.

Die Mischvorrichtung wurde anhand der Zeichnungen an verschiedenen Ausführungsbeispielen beschrieben, die sich im Wesentlichen durch unterschiedliche Ausgestaltung einer Anzeigevorrichtung zur Anzeige einer Druckänderung in der Komponentenkammer unterscheiden. Grundsätzlich kann das Prinzip der erfindungsgemässen Mischvorrichtung mit einer Anzeigevorrichtung für eine Druckänderung in einer Komponentenkammer aber auch weitere Abwandlungen einschliessen. Beispielsweise kann eine zweite Komponente unmittelbar in der Komponentenkammer 3 vorgesehen werden, wobei als Verschlusseinrichtung beispielsweise ein Ventil in der Zuleitung 8 dienen kann. Weiter ist es möglich, die Anzeigevorrichtung an anderer Stelle an der Komponentenkammer 3 vorzusehen, wie beispielsweise in einem Bereich der Umfangswand der Komponentenkammer. Ferner können auch andere Öffnungsmechanismen zur Öffnung eines Komponentenbehälters vorgesehen werden, als diejenigen, die in der oben erwähnten WO 2010/012114A1 beschrieben sind. Ferner kann die Mischkammer auch ohne zusätzliche Mischelemente ausgebildet sein, so dass ein Mischen der Komponenten z. B. durch Schütteln der Mischkammer erfolgen kann. Letztlich können mehr als eine Komponentenkammer vorzugsweise mit je einer Anzeigeeinrichtung an die Mischkammer angeschlossen werden.

### BEZUGSZEICHENLISTE

- 1: Mischkammer
- 2: Erste Komponente
- 3: Komponentenkammer
- 4: Komponentenbehälter
- 5: Zweite Komponente
- 6: Basiseinheit
- 7.: Schraubverbindung
- 8: Zuleitung
- 9: Haltearme/flexible Lippen
- 10: Behälterkopf
- 11: Balg
- 12: Mischer
- 13: Kolbenstange
- 14: Stopfen
- 15: Griff
- 16: Drehhebel
- 17: Mischung
- 18: Ballonbalg
- 19: Äussere Dichtlippe
- 20: Innere Dichtlippe

## Patentansprüche

1. Mischvorrichtung zum Mischen wenigstens zweier Komponenten, die aufweist:
- eine Mischkammer (1) mit einer ersten Komponente (2),
- wenigstens eine Komponentenkammer (3) mit einer zweiten Komponente (5), die mit der Mischkammer (1) über eine Zuleitung (8) verbunden oder verbindbar ist,
- eine Unterdruckeinrichtung, um in der Mischkammer (1) einen Unterdruck zu erzeugen, um dadurch die zweite Komponente von der Komponentenkammer (3) durch die Zuleitung (8) in die Mischkammer (1) zu transportieren, und
- eine Anzeigevorrichtung (11; 18), die an der Komponentenkammer (3) vorgesehen ist, um eine Druckänderung in der Komponentenkammer (3) anzuzeigen, wobei die Anzeigevorrichtung (11; 18) durch einen elastischen Bereich der Komponentenkammer (3) gebildet ist,
**dadurch gekennzeichnet, dass** der elastische Bereich durch einen Balg gebildet ist, der sich an eine Öffnung der Komponentenkammer (3) anschliesst, und dass der Balg als Faltenbalg (11) oder als Ballonbalg (18) ausgebildet ist.

2. Mischvorrichtung nach Anspruch 1, **dadurch gekennzeichnet, dass** die Komponentenkammer (3) ein erstes Ende aufweist, an dem die Zuleitung (8) angeordnet ist, und dass die Komponentenkammer (3) ein zweites Ende aufweist, das dem ersten Ende gegenüberliegt und an dem die Anzeigevorrichtung (11; 18) angeordnet ist.

3. Mischvorrichtung nach Anspruch 2, **dadurch gekennzeichnet, dass** die Komponente (5) oder ein Komponentenbehälter (4) mit der Komponente (5) in der Komponentenkammer (3) zwischen der Zuleitung (8) und der Anzeigevorrichtung (11; 18) gelagert ist.

4. Mischvorrichtung nach einem der vorhergehenden Ansprüche, **dadurch gekennzeichnet, dass** die Anzeigevorrichtung (11; 18) abnehmbar an der Komponentenkammer (3) angeordnet ist.

5. Mischvorrichtung nach einem der vorhergehenden Ansprüche, **dadurch gekennzeichnet, dass** die Anzeigevorrichtung (11; 18) entgegen einer Elastizitätskraft von einer Ausgangsstellung in eine dazu unterschiedliche Anzeigestellung beweglich ist.

6. Mischvorrichtung nach Anspruch 5, **dadurch gekennzeichnet dass** die Anzeigevorrichtung (11; 18) eine Farbmarkierung zur farblichen Unterscheidung der Ausgangsstellung von der Anzeigestellung umfasst.

7. Mischvorrichtung nach einem der vorhergehenden Ansprüche, **dadurch gekennzeichnet, dass** an der Komponentenkammer (3) eine Öffnungseinrichtung (16) zum Öffnen des Komponentenbehälters (4) vorgesehen ist.

8. Mischvorrichtung nach einem der vorhergehenden Ansprüche, **dadurch gekennzeichnet, dass** eine Verschlusseinrichtung (9) vorgesehen ist, die in einer Ausgangsstellung der Mischvorrichtung die Zuleitung (8) blockiert.

9. Mischvorrichtung nach einem der vorhergehenden Ansprüche, **dadurch gekennzeichnet, dass** eine Basiseinheit (6) vorgesehen ist, in der zumindest ein Teil der Zuleitung vorgesehen ist und die Anschlüsse zum Anschliessen der Mischkammer (1) und der wenigstens einen Komponentenkammer (3) an die Zuleitung (8), beziehungsweise den Teil der Zuleitung (8), aufweist.

## Claims

1. A mixing apparatus for mixing at least two components, the mixing apparatus comprising:
- a mixing chamber (1) with a first component (2),
- at least one component chamber (3) with a second component (5), said component chamber being connected or being adapted to be connected to the mixing chamber (1) via a feed line (8),
- a negative pressure device in order to generate a negative pressure in the mixing chamber (1) so as to transport the second component from the component chamber (3) through the feed line (8) into the mixing chamber (1), and
- a display apparatus (11; 18) which is provided on the component chamber (3) in order to indicate a pressure change in the component chamber (3),
**characterized in that** the display apparatus (11; 18) is formed by an elastic region of the component chamber (3) .

2. The mixing apparatus as claimed in claim 1, **characterized in that** the display apparatus (11; 18) is designed to display a change in the volume of the component chamber (3).

3. The mixing apparatus as claimed in either of the preceding claims, **characterized in that** the display apparatus (11; 18) is designed to display a pressure reduction in the component chamber (3).

4. The mixing apparatus as claimed in one of the preceding claims, **characterized in that** the display apparatus (11; 18) is designed to display an emptying of the component (5) from the component chamber (3).

5. The mixing apparatus as claimed in one of the preceding claims, **characterized in that** the component chamber (3) has a first end at which the feed line (8) is arranged, and **in that** the component chamber (3) has a second end which is opposite the first end and on which the display apparatus (11; 18) is arranged.

6. The mixing apparatus as claimed in claim 5, **characterized in that** the component (5) or a component container (4) with the component (5) is arranged in the component chamber (3) between the feed line (8) and the display apparatus (11; 18).

7. The mixing apparatus as claimed in one of the preceding claims, **characterized in that** the display apparatus (11; 18) is movable counter to an elastic force from a starting position into a display position which differs therefrom.

8. The mixing apparatus as claimed in one of the preceding claims, **characterized in that** the elastic region is formed by a bellows (11; 18) which is connected to an opening of the component chamber (3).

9. The mixing apparatus as claimed in claim 8, **characterized in that** the bellows is designed as an expansion bellows (11) or as a balloon bellows (18).

10. The mixing apparatus as claimed in one of the preceding claims, **characterized in that** the display apparatus (11; 18) is arranged removably on the component chamber (3).

11. The mixing apparatus as claimed in one of the preceding claims, **characterized in that** the display apparatus (11; 18) comprises a color marking for differentiation of the starting position from the display position by color.

12. The mixing apparatus as claimed in one of the preceding claims, **characterized in that** an opening device (16) for opening the component container (4) is provided on the component chamber (3).

13. The mixing apparatus as claimed in one of the preceding claims, **characterized in that** a closure device (9), which blocks the feed line (8) in a starting position of the mixing apparatus, is provided.

14. The mixing apparatus as claimed in one of the preceding claims, **characterized in that** a base unit (6) is provided, in which at least one part of the feed line is provided and which has connections for connecting the mixing chamber (1) and the at least one component chamber (3) to the feed line (8) or to the part of the feed line (8).

## Revendications

1. Dispositif mélangeur pour le mélange d'au moins deux composants, présentant :
- une cellule de mélange (1) contenant un premier composant (2),
- au moins une cellule à composant (3) contenant un second composant (5) et qui est reliée ou peut être reliée à la cellule de mélange (1) par une conduite d'alimentation (8),
- un dispositif de dépression permettant de générer une dépression dans la cellule de mélange (1) afin de transporter ainsi le second composant de la cellule à composant (3) à travers la conduite d' alimentation (8) vers la cellule de mélange (1) et
- un dispositif d'affichage (11 ; 18) qui est prévu au niveau de la cellule à composant (3) pour afficher un changement de pression dans la cellule à composant (3), le dispositif d'affichage (11 ; 18) étant constitué par une partie élastique de la cellule à composant (3) ,
**caractérisé en ce que** la partie élastique est formée d'un soufflet qui se raccorde à l'orifice de la cellule à composant (3) et que le soufflet se présente sous forme d'un soufflet accordéon (1) ou d'un soufflet ballon (18).

2. Dispositif mélangeur selon la revendication 1, **caractérisé en ce que** la cellule à composant (3) présente une première extrémité au niveau de laquelle la conduite d'alimentation (8) est disposée et que la cellule à composant (3) présente une seconde extrémité qui est opposée à la première extrémité et au niveau de laquelle le dispositif d'affichage (11 ; 18) est disposé.

3. Dispositif mélangeur selon la revendication 2, **caractérisé en ce que** le composant (5) ou un réservoir à composant (4) s'appuie par le composant (5) dans la cellule à composant (3) entre la conduite d'alimentation (8) et le dispositif d'affichage (11 ; 18).

4. Dispositif mélangeur selon une des revendications précédentes, **caractérisé en ce que** le dispositif d'affichage (11 ; 18) est disposé de manière amovible au niveau de la cellule à composant (3).

5. Dispositif mélangeur selon une des revendications précédentes, **caractérisé en ce que** le dispositif d'affichage (11 ; 18) est mobile à l'inverse d'une force d'élasticité depuis une position initiale jusqu'à une position d'affichage en différant.

6. Dispositif mélangeur selon la revendication 5, **caractérisé en ce que** le dispositif d'affichage (11 ; 18) comprend un marquage coloré pour différencier la position initiale de la position d'affichage par la couleur.

7. Dispositif mélangeur selon une des revendications précédentes, **caractérisé en ce qu'**un système d'ouverture est prévu au niveau de la cellule à composant (3) pour ouvrir le réservoir à composant (4).

8. Dispositif mélangeur selon une des revendications précédentes, **caractérisé en ce qu'**un système de fermeture (9) qui bloque la conduite d'alimentation (8) dans une position initiale du dispositif mélangeur est prévu.

9. Dispositif mélangeur selon une des revendications précédentes, **caractérisé en ce qu'**est prévue une unité de base (6) dans laquelle est installée au moins une partie de la conduite d' alimentation et qui présente des raccordements permettant de raccorder l'au moins une cellule à composant (3) et la conduite d'alimentation à la conduite d'alimentation (8) ou la partie de la conduite d'alimentation (8).
